# EUROPEAN PATENT SPECIFICATION

(11) **EP 1 515 705 B1**
(45) Date of publication and mention of the grant of the patent: **21.06.2006**
(21) Application number: 03737141.6
(22) Date of filing: 16.06.2003
(51) Int. Cl.: A61K 9/70, A61K 31/357, A61K 47/34, C08G 77/24

(54) **FLUOROSILOXANE MATRIX CONTROLLED DIFFUSION DRUG DELIVERY SYSTEM**
DIFFUSIONSKONTROLLIERTES WIRKSTOFFABGABESYSTEM MIT FLUOROSILOXAN-MATRIX
SYSTEME D'APPORT DE MEDICAMENT A DIFFUSION REGULEE A MATRICE DE FLUOROSILOXANE

(30) Priority: 19.06.2002 US 175716
(43) Date of publication of application: 23.03.2005
(73) Proprietor: BAUSCH & LOMB INCORPORATED, Rochester, New York 14604-2701 (US)
(72) Inventor: SALAMONE, Joseph, C., Fairport, NY 14450 (US); KUNZLER, Jay, F., Canadaigua, NY 14424 (US); AMMON, Daniel, M., Jr., Rochester, NY 14609 (US)
(74) Representative: Vossius & Partner
(86) International application number: PCT/US2003/019026
(87) International publication number: WO 2004/000288

(56) References cited:
- WO-A-97/20851
- US-A- 5 321 108
- US-A- 5 908 906

## Description

The present invention relates to copolymers useful in the manufacture of matrix controlled diffusion drug delivery systems. More particularly, the present invention relates to matrix controlled diffusion drug delivery systems produced using one or more fluorinated side-chain siloxane polymers.

Conventional drug delivery involving frequent periodic dosing is not Ideal or practical in many instances. For example, with more toxic drugs, conventional periodic dosing can result in high initial drug levels at the time of dosing, followed by low drug levels between doses often times below levels of therapeutic value. Likewise, conventional periodic dosing may not be practical or therapeutically effective in certain instances such as with pharmaceutical therapies targeting the inner eye or brain, due to inner eye and brain blood barriers.

During the last two decades, significant advances have been made in the design of controlled release drug delivery systems. Such advances have been made in an attempt to overcome some of the drug delivery shortcomings noted above. In general, controlled release drug delivery systems include both sustained drug delivery systems designed to deliver a drug for a predetermined period of time, and targeted drug delivery systems designed to deliver a drug to a specific area or organ of the body. Sustained and/or targeted controlled release drug delivery systems may vary considerably by mode of drug release within three basic drug controlled release categories. Basic drug controlled release categories include diffusion controlled release, chemical erosion controlled release and solvent activation controlled release. In a diffusion controlled release drug delivery system, a drug is surrounded by an inert barrier and diffuses from an inner reservoir, or a drug is dispersed throughout a polymer and diffuses from the polymer matrix. In a chemical erosion controlled release drug delivery system, a drug is uniformly distributed throughout a biodegradable polymer. The biodegradable polymer is designed to degrade as a result of hydrolysis to then uniformly release the drug. In a solvent activation controlled release drug delivery system, a drug is immobilized on polymers within a drug delivery system. Upon solvent activation, the solvent sensitive polymer degrades or swells to release the drug. Unfortunately, controlled release drug delivery systems to date do not provide a means by which one may manipulate and control drug delivery systems' drug release rate for specific drugs over a broad range of drugs.

Because of the noted shortcomings of current controlled release drug delivery systems, a need exists for controlled release drug delivery systems that allow for manipulation and control of drug release rates depending on the drug to be delivered, the location of delivery, the purpose of delivery and/or the therapeutic requirements of the individual patient.

US-A-5,908,906 discloses monomeric units useful in reducing the modulus of hydrogels. Silicone hydrogels including the subject monomeric units are said to be especially useful in the formation of biomedical articles such as silicone hydrogel contact lenses. Specific examples of such monomeric unit includes those represented by the following formula: 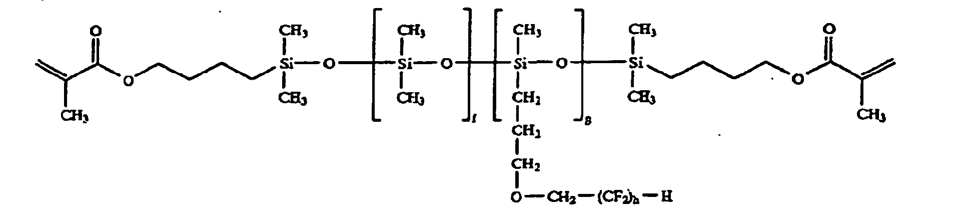 wherein f and g are intergers from 0 to 1000, f+g equals an integer from 2 to 1000, and h is an integer from 1 to about 20.

Novel matrix controlled diffusion drug delivery systems of the present invention, produced from the polymerization of one or more fluorinated side-chain siloxane monomers, allow for manipulation and control of drug release rates depending on the drug to be delivered, the location of delivery, the purpose of delivery and/or the therapeutic requirements of the individual patient. Novel monomers useful in the production of the subject matrix controlled diffusion drug delivery systems are methacrylate-capped polydimethylsiloxanes possessing at

least one perfluorinated side chain. The perfluorinated side chain contains a terminal -CF₂-H functionality. The -CF₂-H functionality of the side chain is extremely versatile for matrix controlled diffusion drug delivery applications. The molecular weight and degree of fluoro-substitution may be varied and the fluorosiloxane monomers can be copolymerized with a wide variety of monomers. Such variability allows for the design of materials possessing a wide range of desirable physical characteristics or properties. At the same time, the terminal -CF₂-H functionality provides for improved solubility characteristics. Improved solubility characteristics allows for improved solubility of the fluorosiloxane monomer with a wide variety of hydrophilic monomers and drugs containing hydrogen-bonding groups.

Accordingly, it is an object of the present invention to provide biocompatible matrix controlled diffusion drug delivery systems.

Another object of the present invention is to provide matrix controlled diffusion drug delivery systems that allow for manipulation and control of drug release rates.

Another object of the present invention is to provide matrix controlled diffusion drug delivery systems that allow for manipulation and control of drug release rates depending on the drug to be delivered.

Another object of the present invention is to provide matrix controlled diffusion drug delivery systems that allow for manipulation and control of drug release rates depending on the location of delivery within the body.

Another object of the present invention is to provide matrix controlled diffusion drug delivery systems that allow for manipulation and control of drug release rates depending on the purpose of drug delivery.

Still another object of the present invention is to provide matrix controlled diffusion drug delivery systems that allow for manipulation and control of drug release rates depending on the therapeutic requirements of the individual patient.

These and other objectives and advantages of the present invention, some of which are specifically described and others that are not, will become apparent from the detailed description and claims that follow.

The present invention provides a matrix controlled diffusion drug delivery system comprising: a fluorinated side-chain siloxane copolymer polymerized with a therapeutically effective amount of at least one pharmaceutically active agent and optionally one or more monomers.

Furthermore, the present invention relates to a matrix controlled diffusion drug delivery system comprising: a fluorinated side-chain siloxane copolymer represented by 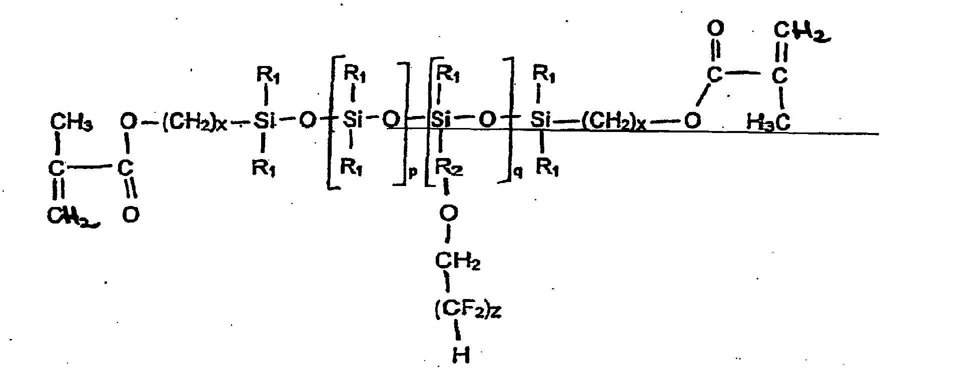 wherein the R₁ groups may be the same or different selected from the group consisting of C₁₋₇alkyl and C₆₋₁₀ aryl; the R₂ group is a C₁₋₇ alkylen; x is a natural number less than 26; p and q may be the same or different natural numbers less than 100 and z is a natural number less than 11, polymerized with a therapeutically effective amount of at least one pharmaceutically active agent and optionally one or more monomers.

In addition, the present invention provides a method of making a matrix controlled diffusion drug delivery system comprising polymerizing a fluorinated side-chain siloxane copolymer with a therapeutically effective amount of at least one pharmaceutically active agent and optionally one or more monomers.

The present invention also provides a method of making a matrix controlled diffusion drug delivery system comprising: polymerizing a fluorinated side-chain siloxane copolymer as defined above with a therapeutically effective amount of at least one pharmaceutically active agent and optionally one or more monomers.

In addition, the present invention relates to a method of making a matrix controlled diffusion drug delivery system comprising: preparing a methacrylate-capped siloxane with a perfluorinated side-chain copolymer; copolymerizing said methacrylate-capped siloxane with a perfluorinated side-chain copolymer with one or more monomers and a therapeutically effective amount of at least one pharmaceutical active agent.

The present invention further provides a matrix controlled diffusion drug delivery system as defined above for use in a method comprising: creating an incision within an eye and implanting said matrix controlled diffusion drug delivery system within said eye through said incision.

The present invention also relates to novel fluorosiloxane monomers useful in the manufacture of novel matrix controlled diffusion drug delivery systems. The subject matrix controlled diffusion drug delivery systems allow for manipulation and control of drug release rates, which may be based on the drug to be delivered, the location of delivery, the purpose of delivery and/or the therapeutic requirements of the individual patient.

The novel fluorosiloxane monomers of the present invention are methacrylate-capped polydimethylsiloxanes possessing at least one perfluorinated side chain. The perfluorinated side chain contains a terminal -CF₂-H functionality that is extremely versatile for drug delivery applications. The fluorosiloxane monomers of the present invention are generally represented by Formula 1 below: 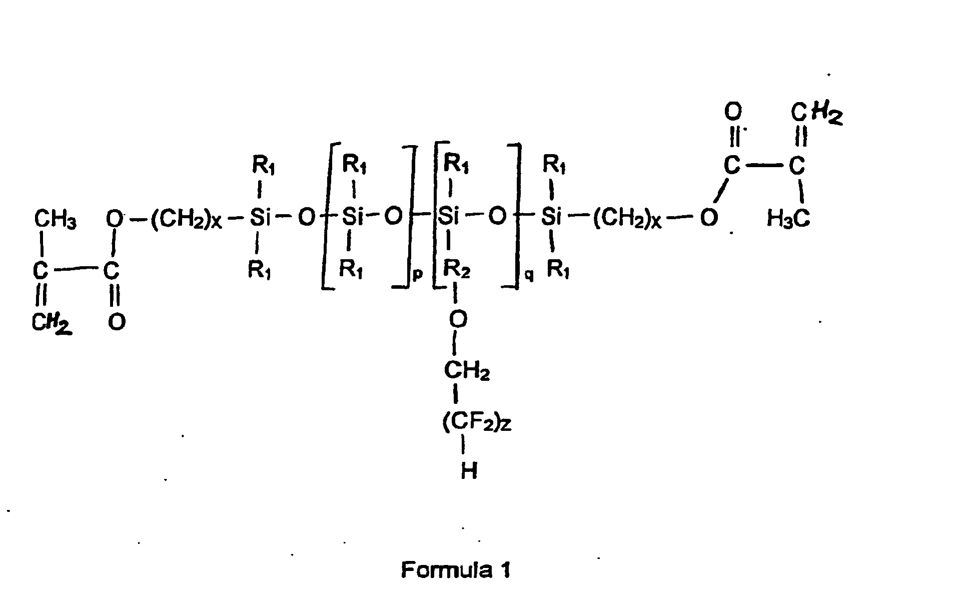 wherein the R₁ groups may be the same or different selected from the group consisting of C₁₋₇ alkyl such as for example but not limited to methyl, propyl or butyl but preferably methyl for improved biocompatability, and C₆₋₁₀ aryl such as for example but not limited to phenyl; the R₂ group is a C₁₋₇ alkylene such as for example but not limited to methylene, ethylene or heptylene but preferably propylene; x is a natural number less than 26; p and q may be the same or different natural numbers less than 100 and z is a natural number less than 11,

with the proviso that compounds of the following formula are excluded: 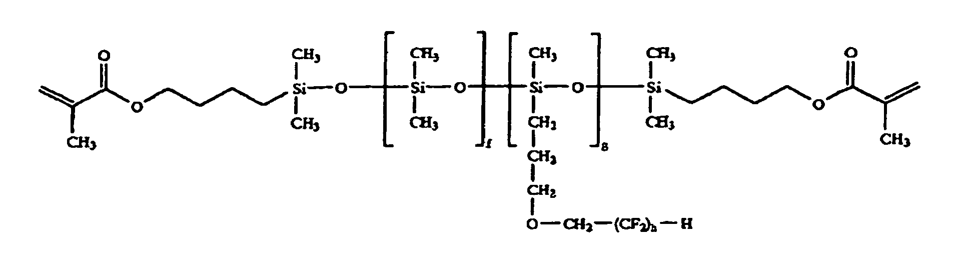 wherein f and g are natural numbers less than 100; and h is a natural number less than 11.

Fluorinated side-chain siloxane monomers of the present invention may be synthesized as represented in Scheme 1 below:

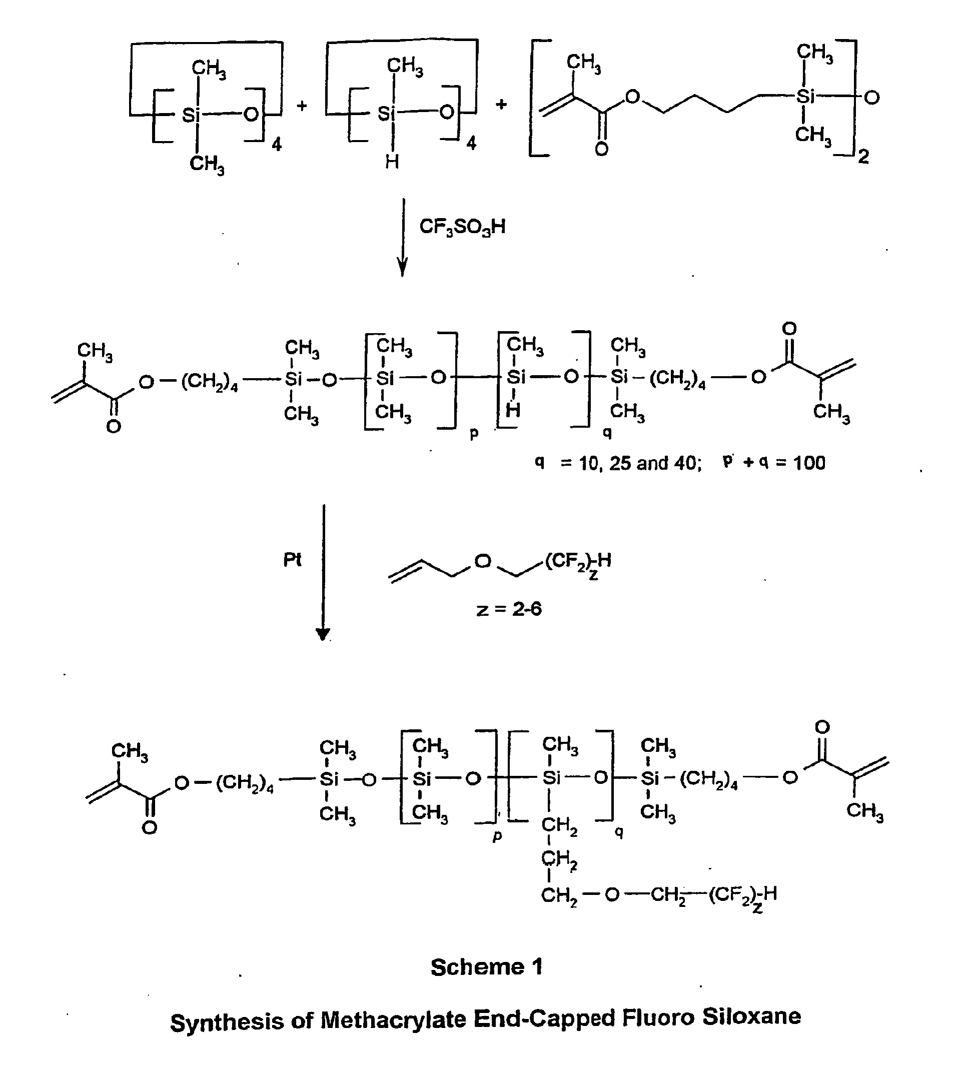

One or more fluorinated side-chain siloxane monomers of the present invention produced as described above may be combined with one or more pharmaceutically active agents and polymerized and/or copolymerized with other monomers. By controlling the concentration of the hydrophobic siloxane backbone, the polar -CF₂-H tail, and any comonomer(s), if used, a particular hydrophobic/hydrophilic balance of characteristics or properties is achieved. The hydrophobic/hydrophilic balance of characteristics may likewise be manipulated to achieve the desired rate of drug release. The desired rate of drug release may be determined based on the drug to be delivered, the location of delivery, the purpose of delivery and/or the therapeutic requirements of the individual patient. The hydrophobic/hydrophilic balance of characteristics dictates the solubility of the drug, and is a primary factor controlling the rate of drug release. In some cases, the polar-CF₂-H tail may be used to hydrogen bond with drugs containing polar groups to decrease the rate of drug release.

Pharmaceutically active agents or drugs useful in the matrix controlled diffusion drug delivery system of the present invention include for example but are not limited to anti-glaucoma agents such as for example but not limited to the beta blockers timolol maleate, betaxolol and metipranolol, mitotics such as for example but not limited to pilocarpine, acetylcholine chloride, isofluorophate, demacarium bromide, echothiophateiodide, phospholine iodide, carbachol and physostigimine, epinephrine and salts such as for example but not limited to dipivefrin hydrochloride, dichlorphenamide, acetazolamide and methazolamide, anti-cataract and anti-diabetic retinopathy agents such as for example but not limited to the aldose reductase inhibitors tolrestat, lisinopril, enalapril and statil, thiol cross-linking agents, anticancer agents such as for example but not limited to retinoic acid, methotrexate, adriamycin, bleomycin, triamcinoline, mitomycin, cisplatinum, vincristine, vinblastine, actinomycin-D, ara-c, bisantrene, activated cytoxan, melphalan, mithramycin, procarbazine and tamoxifen, immune modulators, anti-clotting agents such as for example but not limited to tissue plasminogen activator, urokinase and streptokinase, anti-tissue damage agents such as for example but not limited to superoxide dismutase, proteins and nucleic acids such as for example but not limited to mono- and poly-clonal antibodies, enzymes, protein hormones and genes, gene fragments and plasmids, steroids, particularly anti-inflammatory or anti-fibrous agents such as for example but not limited to loteprednol, etabonate, cortisone, hydrocortisone, prednisolone, prednisome, dexamethasone, progesterone-like compounds, medrysone (HMS) and fluorometholone, non-steroidal anti-inflammatory agents such as for example but not limited to ketrolac tromethamine, dichlofenac sodium and suprofen, antibiotics such as for example but not limited to loridine (cephaloridine), chloramphenicol, clindamycin, amikacin, tobramycin, methicillin, lincomycin, oxycillin, penicillin, amphotericin B, polymyxin B, cephalosporin family, ampicillin, bacitracin, carbenicillin, cepholothin, colistin, erythromycin, streptomycin, neomycin, sulfacetamide, vancomycin, silver nitrate, sulfisoxazole diolamine and tetracycline, other antipathogens including anti-viral agents such as for example but not limited to idoxuridine, trifluorouridine, vidarabine (adenine arabinoside), acyclovir (acycloguanosine), pyrimethamine, trisulfapyrimidine-2, clindamycin, nystatin, flucytosine, natamycin, and miconazole, piperazine derivatives such as for example but not limited to diethylcarbamazine, and cycloplegic and mydriatic agents such as for example but not limited to atropine, cyclogel, scopolamine, homatropine and mydriacyl.

Other pharmaceutical agents or drugs include anticholinergics, anticoagulants, antifibrinolytics, antihistamines, antimalarials, antitoxins, chelating agents, hormones, immunosuppressives, thrombolytics, vitamins, salts, desensitizers, prostaglandins, amino acids, metabolites and antiallergenics.

Pharmaceutical agents or drugs of particular interest include hydrocortisone (5-20 mcg/l as plasma level), gentamycin (6-10 mcg/ml in serum), 5-fluorouracil (∼30 mg/kg body weight in serum), sorbinil, interleukin-2, phakan-a (a component of glutathione), thioloa-thiopronin, bendazac, acetylsalicylic acid, trifluorothymidine, interferon (α, β and γ), immune modulators such as for example but not limited to lymphokines and monokines and growth factors.

Monomers useful for copolymerization with the fluorinated side-chain siloxane monomers of the present invention and one or more pharmaceutically active agents include for example but are not limited to methyl methacrylate, N,N-dimethylacrylamide, acrylamide, N-methylacrylamide, 2-hydroxyethyl methacrylate, hydroxyethoxyethyl methacrylate, hydroxydiethoxyethyl methacrylate, methoxyethyl methacrylate, methoxyethoxyethyl methacrylate, methoxydiethoxyethyl methacrylate, poly(ethylene glycol) methacrylate, methoxy-poly(ethylene glycol) methacrylate, methacrylic acid, sodium methacrylate, glycerol methacrylate, hydroxypropyl methacrylate, N-vinylpyrrolidione and hydroxybutyl methacrylate.

The subject matrix controlled diffusion drug delivery systems of the present invention produced using one or more fluorinated side-chain siloxane monomers are described in still greater detail in the examples that follow.

### EXAMPLE 1: Synthesis of methacrylate end-capped poly (25 mole percent methyl siloxane)-co-(75 mole percent dimethylsiloxane) (M₂D₇₅D₂₅H)

To a 100 mL round bottom flask under dry nitrogen was added D₄ (371.9 g, 1.25 mole), D₄H (100.4 g, 0.42 mole) and M₂ (27.7 g, 0.7 mole). Trifluoromethane sulfonic acid (0.25 percent, 1.25 g, 8.3 mmole) was added as initiator. The reaction mixture was stirred 24 hours with vigorous stirring at room temperature. Sodium bicarbonate (10 g, 0.119 mole) was then added and the reaction mixture was again stirred for 24 hours. The resultant solution was filtered through a 0.3 µ Teflon™ (E.I. DuPont De Nemours & Co., Wilmington, Delaware) filter. The filtered solution was vacuum stripped and placed under vacuum (>0.1 mm Hg) at 50° Celsius to remove the unreacted silicone cyclics. The resulting silicone hydride functionalized siloxane was a viscous, clear fluid. Yield 70 percent; SEC: Mn=7,500, Mw/Mn=2.2; ¹H-NMR(CDCl₃, TMS, δ, ppm): 0.1 (s, 525H, Si-CH₃), 0.5 (t, 4H, Si-CH₂-), 1.5-1.8 (m, 8H, Si-CH₂-CH₂-CH₂ and Si-CH₂-CH₂-CH₂), 1.95 (s, 6H, =C-CH₃), 4.1 (t, 4H, -CH₂-O-C(O)), 4.5 (s, 25H, Si-H), 5.6 (s, 2H, =C-H) and 6.2 (s, 2H, =C-H).

### EXAMPLE 2: Synthesis of methacrylate end-capped poly (25 mole percent (3-(2,2,3,3,4,4,5,5,-octafluoropentoxy)propyl methyl siloxane)-co-(75 mole percent dimethylsiloxane)

To a 500 mL round bottom flask equipped with a magnetic stirrer and water condenser was added M₂D₇₅D₂₅H (15 g, 0.002 mole), allyloxyoctafluoropentane (27.2 g, 0.1 mole), tetramethyldisiloxane platinum complex (2.5 mL of a 10 percent solution in xylenes), 75 mL of dioxane and 150 mL of anhydrous tetrahydrofuran under a nitrogen blanket. The reaction mixture was heated to 75° Celsius and the reaction was monitored by IR and ¹H-NMR spectroscopy for loss of silicone hydride. The reaction was complete in 4 to 5 hours of reflux. The resulting solution was placed on a rotoevaporator to remove tetrahydrofuran and dioxane. The resultant crude product was diluted with 300 mL of a 20 percent methylene chloride in pentane solution and passed through a 15 gram column of silica gel using a fifty percent solution of methylene chloride in pentane as eluant. The collected solution was again placed on the rotoevaporator to remove solvent and the resultant clear oil was placed under vacuum (<0.1 mm Hg) at 50° Celsius for four hours. The resulting octafluoro functionalized side-chain siloxane was a viscous, clear fluid. Yield 65 percent;
SEC: Mn=18,000, Mw/Mn=2.3; ¹H-NMR (CDCl₃, TMS, δ, ppm): 0.1 (s, 525H, Si-CH₃), 0.5 (t, 54H, Si-CH₂-), 1.5-1.8 (m, 58H, Si-CH₂-CH₂-CH₂ and Si-CH₂₋CH₂-CH₂), 1.95 (s, 6H, =C-CH₃), 4.1 (t, 4H, -CH₂-O-C(O)), 5.6 (s, 2H, =C-H), 5.8 (t, 17H, -CF₂-H), 6.1 (m, 35H, -CF₂-H and =C-H) and 6.3 (t, 17 H, -CF₂₋H).

### EXAMPLE 3: Casting of film

A film was cast using 70 parts of a methacrylate end-capped DP 100 polydimethylsiloxane containing 25 mole percent of the octafluoropropyloxy side-chain, 30 parts of dimethyl acrylamide, 0.5 percent Darocur™ 1173 (Ciba-Geigy, Basel, Switzerland) and 5 percent by weight of the drug Fluocinolone Acetonide (FA). The cure conditions consisted of a two hour ultraviolet irradiation. The film was extracted in isopropanol for 24 hours, air dried and then hydrated in a borate buffered saline. The resultant film possessed a modulus of 170 g/mm², a tear of 3 g/mm and a water content of
30.0 percent by weight.

### EXAMPLE 4: Casting of film

A film was cast using 30 parts of a methacrylate end-capped DP 100 polydimethylsiloxane containing 25 mole percent of the octafluoropropyloxy side-chain, 70 parts of dimethyl acrylamide, 0.5 percent Darocur™ 1173 and 5 percent by weight of the drug FA. The cure conditions consisted of a two hour ultraviolet irradiation. The film was extracted in isopropanol for 24 hours followed by a vacuum dry to remove the isopropanol.

### EXAMPLE 5: Preparation of diffusion controlled release drug delivery system

A 10 mm disc of film from each Example 3 and Example 4 was prepared and mounted to a Kontes diffusion cell between a solution of pH 4 acetate buffer. The film from Example 3 is hereinafter referred to as Sample 1 and the film from Example 4 is hereinafter referred to as Sample 2. The rate of drug release was monitored by ultraviolet (UV) techniques at 34° Celsius. The best results to date were for films of Sample 2 consisting of 30 parts of the methacrylate end-capped fluorosiloxane (DP 100, 25 mole percent fluoro side- chain), 70 parts of methyl methacrylate and 5 percent FA. Table 1 and Chart 1 below show the release characteristics of Series 1 and Series 2, which are duplicates of Sample 2, monitored over a period of 1200 hours. For each series
tested, a zero-order linear relationship was established shortly after the initial drug release. Based on this relationship, a constant drug release of 800 days (Series 1) and 1000 days (Series 2) should occur, assuming this linear relationship is maintained.

**Table 1 Drug Release from F-Si/MMA (30/70) Film**

| **Time (hours)** | **Series 1 850 µg** | **Series 2 850 µg** |
|---|---|---|
| 0 | 0 | 0 |
| 1 | 2.4 | 1.9 |
| 35 | 12.1 | 11.6 |
| 119 | 21.9 | 21.0 |
| 244 | 41.6 | 29.2 |
| 340 | 48.7 | 35.6 |
| 508 | 56.4 | 41.5 |
| 676 | 65.3 | 47.8 |
| 844 | 72.9 | 54.2 |
| 1012 | 80.2 | 58.8 |
| 1180 | 86.9 | 63.5 |

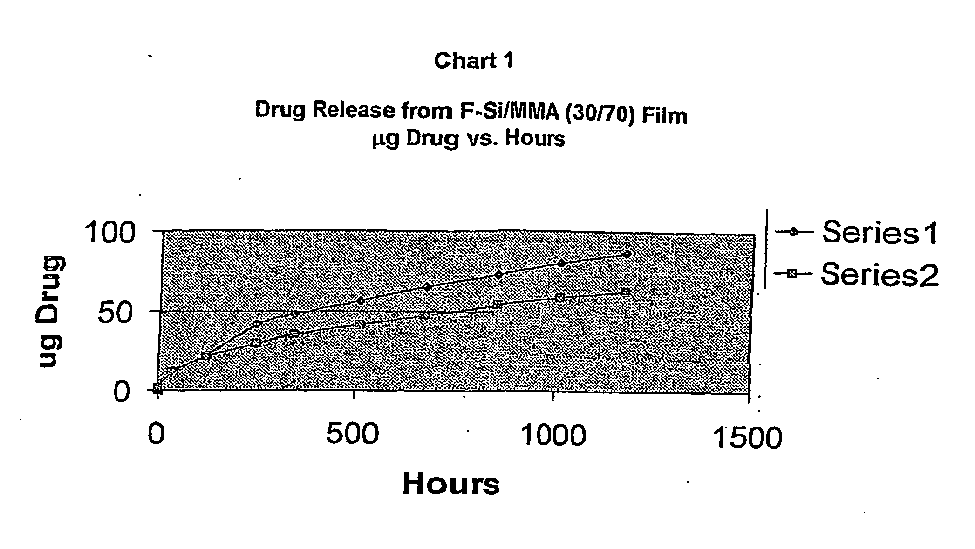

**[0027]** Matrix controlled diffusion drug delivery systems of the present invention may be manufactured in any shape or size suitable for the intended purpose for which they are intended to be used. For example, for use as an inner back of the eye implant, the subject matrix controlled diffusion drug delivery system would preferably be no larger in size than 3 mm². Methods of manufacturing the subject matrix controlled diffusion drug delivery systems includes cast molding, extrusion, and like methods known to those skilled in the art. Once manufactured, the subject matrix controlled diffusion drug delivery systems are packaged and sterilized using customary methods known to those skilled in the art.

Matrix controlled diffusion drug delivery systems of the present invention may be used in a broad range of therapeutic applications. In the field of ophthalmology for example, the subject controlled release drug delivery system is used by implantation within the interior portion of an eye. However, the subject matrix controlled diffusion drug delivery system may likewise be used in accordance with other surgical procedures known to those skilled in the field of ophthalmology.

## Claims

1. A matrix controlled diffusion drug delivery system comprising:
a fluorinated side-chain siloxane copolymer polymerized with a therapeutically effective amount of at least one pharmaceidically active agent and optionally one or more monomers.

2. A matrix controlled diffusion drug delivery system comprising:
a fluorinated side-chain siloxane copolymer represented by 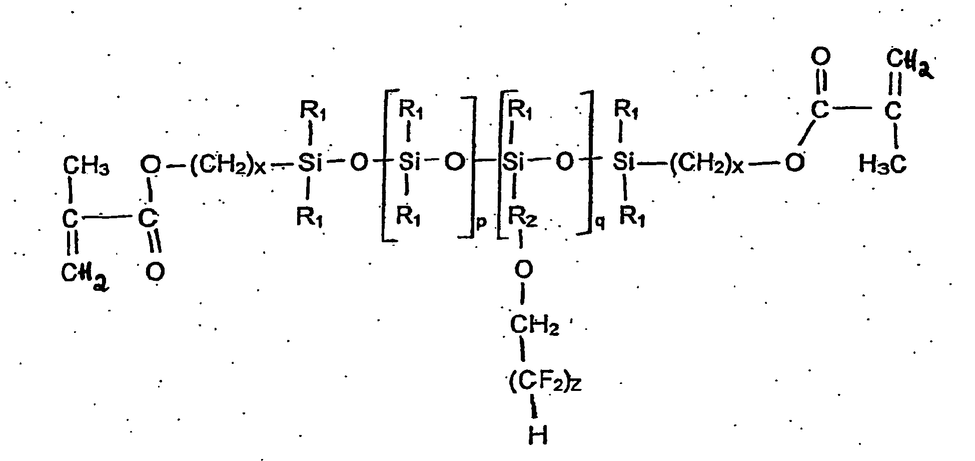
wherein the R₁ groups may be the same or different selected from the group consisting of C₁₋₇ alkyl and C₆₋₁₀ aryl; the R₂ group is a C₁₋₇ alkylene; x is a natural number less than 26; p and q may be the same or different natural numbers less than 100 and z is a natural number less than 11, polymerized with a therapeutically effective amount of at least one pharmaceutically active agent and optiortally one or more monomers.

3. The matrix controlled diffusion drug delivery system of claim 1 or 2 wherein said at least one pharmaceutically active agent is selected. from the group consisting of anti-glaucoma agents, anti-cataract agents, anti-diabetic retinopathy agents, thiol cross-linking agents, anti-cancer agents, immune modulators, anti-clotting agents, anti-tissue damage agents, anti-inflammatory agents, anti-fibrous agents, non-steroidal anti-inflammatory agents, antibiotics, anti-pathogen agents, piperazine derivatives, cycloplegic agents and mydriatic agents.

4. The matrix controlled diffusion drug delivery system of claim 1 or 2 wherein said at least one pharmaceutically active agent is selected from the group consisting of anticholinergics, anticoagulants, antifibrinolyties, antihistamines, antimalarials, antitoxins, chelating agents, hormones, immunosuppressives; thrombolytics, vitamins, salts, desensitizers, prostaglandins, amino acids, metabolites and antiallergenics.

5. The matrix controlled diffusion drug delivery system of claim 1 or 2 wherein said at least one pharmaceutically active agent is selected from the group consisting of hydrocortisone, gentamycin, 5-fluorouracil, sorbinil, interleukin-2, phakan-a, thioloa-thiapronin, bendazac, acetylsalicylic acid, fluocinolone acetonide, trifluorothymidine, interferon, immune modulators and growth factors.

6. The matrix controlled diffusion drug delivery system of claim 1 or 2 wherein said one or more monomers are selected from the group consisting of methyl methacrylate, N,N-dimethylacrylamide, acrylamide, N-methylacrylamide, 2-hydroxyethyl methacrylate, hydroxyethoxyethyl methacrylate, hydroxydiethoxyethyl methacrylate, methoxyethyl methacrylate, methoxyethoxyethyl methacrylate, methoxydiethoxyethyl methacrylates, poly(ethylene glycol) methacrylate, methoxy-poly(ethylene glycol) methacrylate, methacrylic acid, sodium methacrylate, glycerol methacrylate, hydroxypropyl methacrylate, N-vinylpyrrolidone and hydroxybutyl methacrylate.

7. A fluorinated side-chain siloxane copolymer comprising; 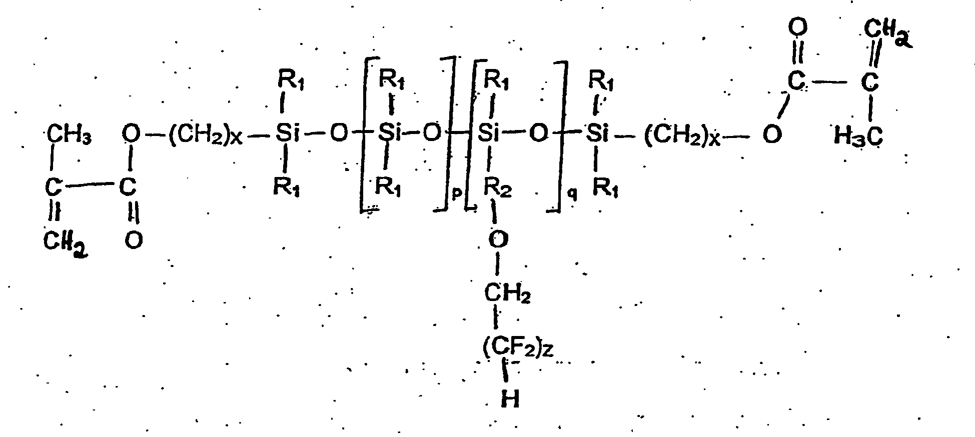 wherein the R₁ groups may be the same or different selected from the group consisting of C₁₋₇ alkyl and C₆₋₁₀ aryl; the R₂ group is a C₁₋₇ alkylene; x is a natural number less than 26; p and q may be the same or different natural numbers less than 100 and z is a natural number less than 11,with the proviso that compounds of the following formula are excluded: 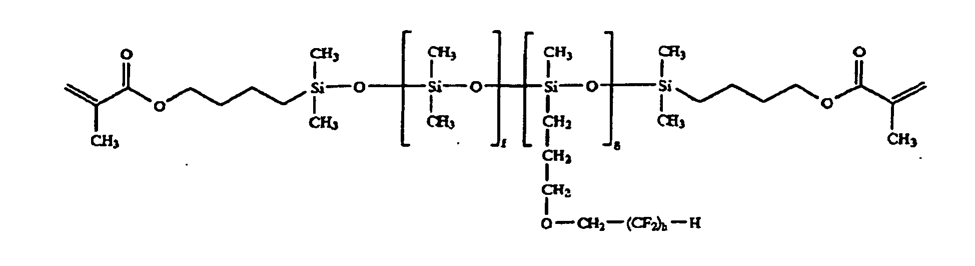 wherein f and g are natural numbers less than 100; and h is a natural number less than 11.

8. A method of making a matrix controlled diffusion drug delivery system comprising:
polymerizing a fluorinated side-chain siloxane copolymer with a therapeutically effective amount of at least one pharmaceutically active agent and optionally one or more monomers.

9. A method of making a matrix controlled diffusion drug delivery system comprising:
polymerizing a fluorinated side-chain siloxane copolymer represented by 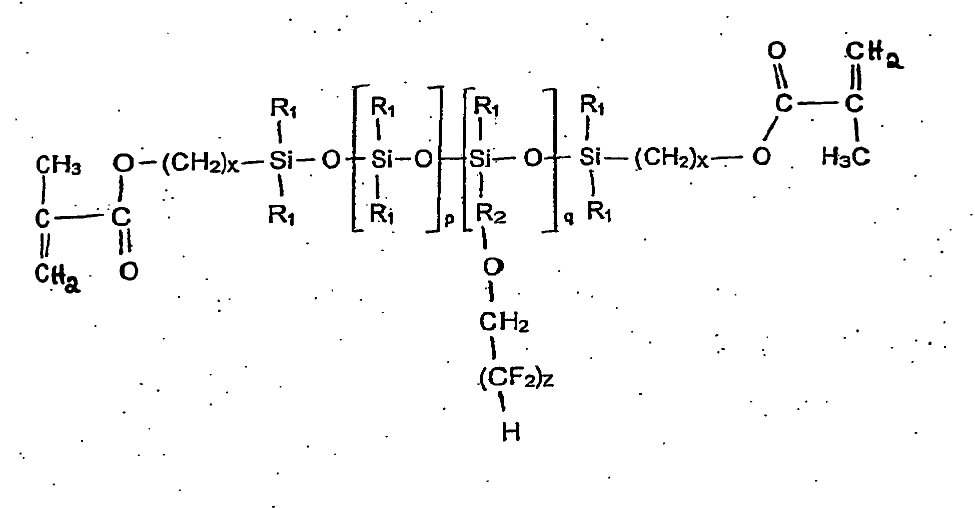
wherein the R₁ groups may be the same or different selected from the group consisting of C₁₋₇ alkyl and C₆₋₁₀ aryl; the R₂ group is a C₁₋₇ alkylene; x is a natural number less than 26; p and q may be the same or different natural numbers less than 100 and z is a natural number less than 11, with a therapeutically effective amount of at least one pharmaceutically active agent and optionally one or more monomers.

10. A method of making a matrix controlled dhfusion drug delivery system comprising:
preparing a methacrylate-capped siloxane with a perfluorinated side-chain copolymer,
copolymerizing said methacrylate-capped siloxane with a perfluorinated side-chain copolymer with one or more monomers and a thempeutically effective amount of at least one pharmaceutically active agent.

11. The method of claim 8, 9 or 10 wherein said at least one pharmaceutically active agent is selected from the group consisting of anti-glaucoma agents, anti-cataract agents, anti-diabetic retinopathy agents, thiol cross-linking agents, anti-cancer agents, immune mediators, anti-clotting agents, anti-tissue damage agents, anti-inflammatory agents, anti-fibrous agents, non-steroidal anti-inflammatory agents, antibiotics, anti-pathogen agents, piperazine derivatives, cycloplegic agents and mydriatic agents.

12. The method of claim 8, 9 or 10 wherein said at least one pharmaceutically active agent is selected from the group consisting of anticholinergics, anticoagulants, antifibrinolytics, antihistamines, antimalarials, antitoxins, chelating agents, hormones, immunosuppressives, thrombolytics, vitamins, salts, desensitizers, prostagiandins, amino acids, metabolites and antiallergenics.

13. The method of claim 8, 9 or 10 wherein said at least one pharmaceutically active agent is selected from the group consisting of hydrocortisone, gentamycin, 5-fluorouracil, sorbinil, interleukin-2, phakan-a, thioloa-thiopronin, bendazac, acetylsalicylic acid, fluocinolone acetonide, trifluorothymidine, interferon, immune modulators and growth factors.

14. The method of claim 8, 9 or 10 wherein said one or more monomers are selected from the group consisting of methyl methacrylate, N,N-dimethylacrylamide, acryiamide, N-methyfacrylamide, 2-hydroxyethyl methacrylate, hydroxyethoxyethyl methacrylate, hydroxydiethoxyethyl methacrylate, methoxyethyl methacrylate, methoxyethoxyethyl methacrylate; methoxydiethoxyethyl methacrylate, poly(ethylene glycol) methacrylate, methoxy-poly(ethylene glycol) methacrylate, methacrylic acid, sodium methacrylate, glycerol methacrylate, hydroxypropyl methacrylate, N-vinylpyrrolidione and hydroxybutyl methacrylate.

15. The matrix controlled diffusion drug delivery system of claim 1 or 2 for use in a method comprising:
creating an incision within an eye and
implanting said matrix controlled diffusion drug delivery system within said eye through said incision.

## Patentansprüche

1. Matrixgesteuertes Arzneistoffdiffusionsabgabesystem, umfassend:
ein Siloxancopolymer mit fluorierter Seitenkette, welches mit einer therapeutisch wirksamen Menge mindestens eines pharmazeutisch wirksamen Mittels und gegebenenfalls einem oder mehreren Monomeren polymerisiert ist.

2. Matrixgesteuertes Arzneistoffdiffusionsabgabesystem, umfassend:
ein Siloxancopolymer mit fluorierter Seitenkette, dargestellt durch 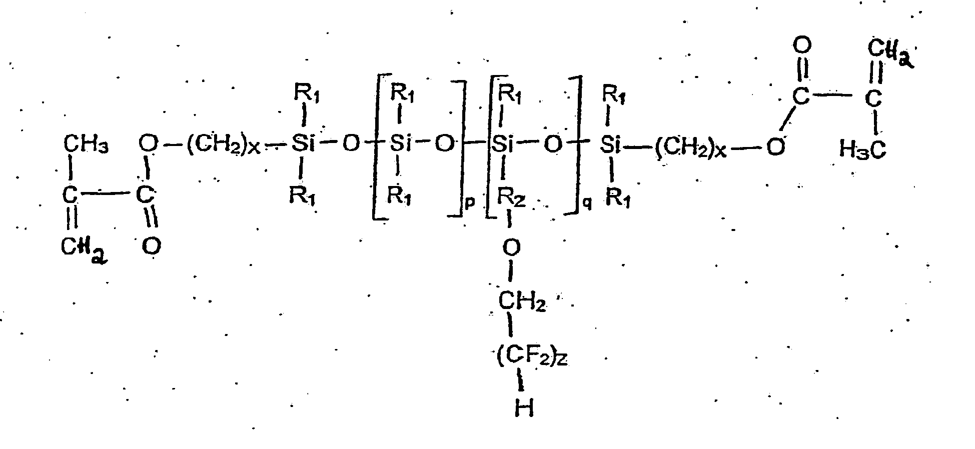
wobei die Reste R₁ gleich oder verschieden sein können und ausgewählt sind aus C₁₋₇₋Alkyl und C₆₋₁₀-Aryl; der Rest R₂ ein C₁₋₇-Alkylen ist; x eine natürliche Zahl kleiner als 26 ist; p und q gleiche oder verschiedene natürliche Zahlen kleiner als 100 sein können und z eine natürliche Zahl kleiner als 11 ist, polymerisiert mit einer therapeutisch wirksamen Menge mindestens eines pharmazeutisch wirksamen Mittels und gegebenenfalls einem oder mehreren Monomeren.

3. Matrixgesteuertes Arzneistoffdiffusionsabgabesystem gemäß Anspruch 1 oder 2, wobei das mindestens eine pharmazeutisch wirksame Mittel ausgewählt ist aus Mitteln gegen Glaukom, Antikataraktika, Mitteln gegen diabetische Rethinopatie, Thiol vernetzenden Mitteln, Mitteln gegen Krebs, Immunmodulatoren, Antigerinnungsmitteln, Mitteln gegen Gewebsverletzungen, entzündungshemmenden Mitteln, antifibrösen Mitteln, nicht-steroidalen entzündungshemmenden Mitteln, Antibiotika, antipathogenen Mitteln, Piperazinderivaten, Mitteln zur Cycloplegie und Pupillen erweiternden Mitteln.

4. Matrixgesteuertes Arzneistoffdiffusionsabgabesystem gemäß Anspruch 1 oder 2, wobei das mindestens eine pharmazeutisch wirksame Mittel ausgewählt ist aus Anticholinergika, Antikoagulanzien, Antifibrinolytika, Antihistaminika, Antimalariamitteln, Antitoxinen, Chelatbildnern, Hormonen, Immunsuppressiva, Thrombolytika, Vitaminen, Salzen, Desensibilierungsmitteln, Prostaglandinen, Aminosäuren, Metaboliten und Antiallergika.

5. Matrixgesteuertes Arzneistoffdiffusionsabgabesystem gemäß Anspruch 1 oder 2, wobei das mindestens eine pharmazeutisch wirksame Mittel ausgewählt ist aus Hydrocortison, Gentamycin, 5-Fluoruracil, Sorbinil, Interleukin-2, Phakan-a, Thioloa-Thiopronin, Bendazac, Acetylsalicylsäure, Fluocinolonacetonid, Trifluorthymidin, Interferon, Immunmodulatoren und Wachstumsfaktoren.

6. Matrixgesteuertes Arzneistoffdiffusionsabgabesystem gemäß Anspruch 1 oder 2, wobei das eine oder die mehreren Monomere ausgewählt sind aus Methylmethacrylat, N,N-Dimethylacrylamid, Acrylamid, N-Methylacrylamid, 2-Hydroxyethylmethacrylat, Hydroxyethoxyethylmethacrylat, Hydroxydiethoxyethylmethacrylat, Methoxyethylmethacrylat, Methoxyethoxyethylmethacrylat, Methoxydiethoxyethyhnethacrylat, Poly(ethylenglykol)methacrylat, Methoxy-poly(ethylenglykol)methacrylat, Methacrylsäure, Natriummethacrylat, Glyzerinmethacrylat, Hydroxypropylmethacrylat, N-Vinylpyrrolidion und Hydroxybutylmethacrylat.

7. Siloxancopolymer mit fluorierter Seitenkette, umfassend: 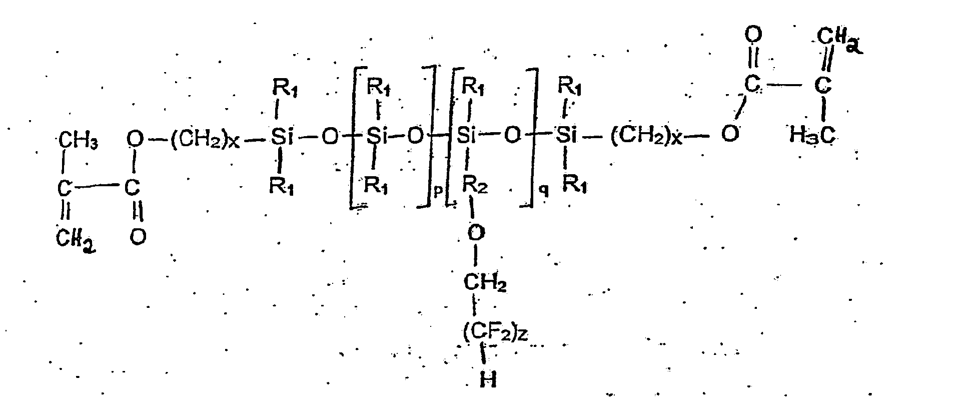 wobei die Reste R₁ gleich oder verschieden sein können und ausgewählt sind aus C₁₋₇₋Alkyl und C₆₋₁₀-Aryl; der Rest R₂ ein C₁₋₇-Alkylen ist; x eine natürliche Zahl kleiner als 26 ist; p und q gleiche oder verschiedene natürliche Zahlen kleiner als 100 sein können und z eine natürliche Zahl kleiner als 11 ist, mit der Maßgabe, dass Verbindungen der folgenden Formel ausgeschlossen sind: 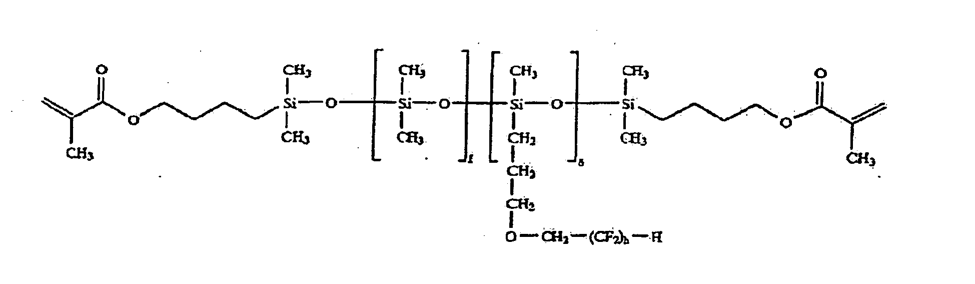 wobei f und g natürliche Zahlen kleiner als 100 sind; und h eine natürliche Zahl kleiner als 11 ist.

8. Verfahren zur Herstellung eines matrixgesteuerten Arzneistoffdiffusionsabgabesystems, umfassend:
Polymerisieren eines Siloxancopolymers mit fluorierter Seitenkette mit einer therapeutisch wirksamen Menge mindestens eines pharmazeutisch wirksamen Mittels und gegebenenfalls einem oder mehreren Monomeren.

9. Verfahren zur Herstellung eines matrixgesteuerten Arzneistoffdiffusionsabgabesystems, umfassend:
Polymerisieren eines Siloxancopolymers mit fluorierter Seitenkette, dargestellt durch 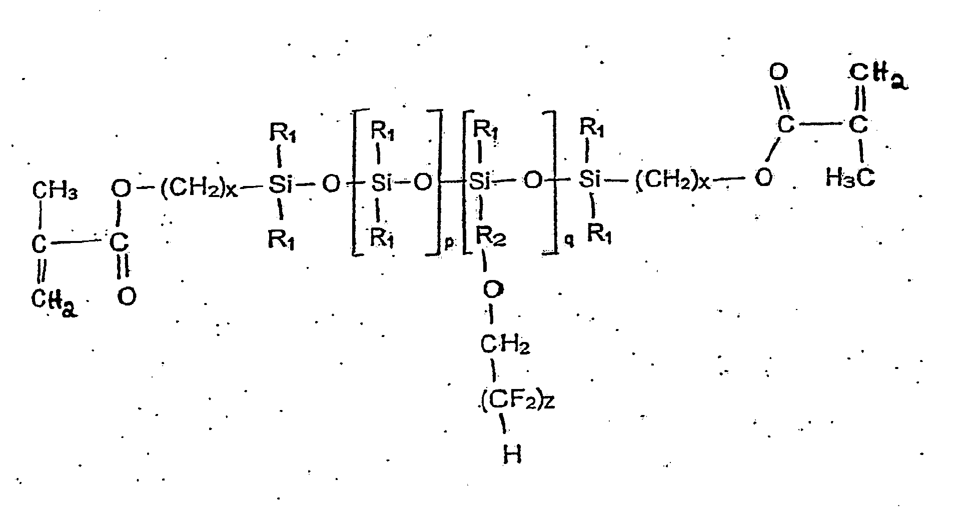
wobei die Reste R₁ gleich oder verschieden sein können und ausgewählt sind aus C₁₋₇₋Alkyl und C₆₋₁₀-Aryl; der Rest R₂ ein C₁₋₇-Alkylen ist; x eine natürliche Zahl kleiner als 26 ist; p und q gleiche oder verschiedene natürliche Zahlen kleiner als 100 sein können und z eine natürliche Zahl kleiner als 11 ist, mit einer therapeutisch wirksamen Menge mindestens eines pharmazeutisch wirksamen Mittels und gegebenenfalls einem oder mehreren Monomeren.

10. Verfahren zur Herstellung eines matrixgesteuerten Arzneistoffdiffusionsabgabesystems, umfassend:
Herstellen eines mit endständigem Methacrylat versehenen Siloxancopolymers mit perfluorierter Seitenkette;
Copolymerisieren des mit endständigem Methacrylat versehenen Siloxancopolymers mit perfluorierter Seitenkette mit einem oder mehreren Monomeren und einer therapeutisch wirksamen Menge mindestens eines pharmazeutisch wirksamen Mittels.

11. Verfahren gemäß Anspruch 8, 9 oder 10, wobei das mindestens eine pharmazeutisch wirksame Mittel ausgewählt ist aus Mitteln gegen Glaukom, Antikataraktika, Mitteln gegen diabetische Retinopathie, Thiol vernetzenden Mitteln, Mitteln gegen Krebs, Immunmodulatoren, Antigerinnungsmitteln, Mitteln gegen Gewebsverletzungen, entzündungshemmenden Mitteln, antifibrösen Mitteln, nicht-steroidalen entzündungshemmenden Mitteln, Antibiotika, antipathogenen Mitteln, Piperazinderivaten, Mitteln zur Cycloplegie und Pupillen erweiternden Mitteln.

12. Verfahren gemäß Anspruch 8, 9 oder 10, wobei das mindestens eine pharmazeutisch wirksame Mittel ausgewählt ist aus Anticholinergika, Antikoagulanzien, Antifibrinolytika, Antihistaminika, Antimalariamitteln, Antitoxinen, Chelatbildnern, Hormonen, Immunsuppressiva, Thrombolytika, Vitaminen, Salzen, Desensibilierungsmitteln, Prostaglandinen, Aminosäuren, Metaboliten und Antiallergika.

13. Verfahren gemäß Anspruch 8, 9 oder 10, wobei das mindestens eine pharmazeutisch aktive Mittel ausgewählt ist aus Hydrocortison, Gentamycin, 5-Fluoruracil, Sorbinil, Interleukin-2, Phakan-a, Thioloa-Thiopronin, Bendazac, Acetylsalicylsäure, Fluocinolonacetonid, Trifluorthymidin, Interferon, Immunmodulatoren und Wachstumsfaktoren.

14. Verfahren gemäß Anspruch 8, 9 oder 10, wobei das eine Monomer oder die mehreren Monomere ausgewählt sind aus Methylmethacrylat, N,N-dimethylacrylamid, Acrylamid, N-Methylacrylamid, 2-Hydroxyethyhmethacrylat, Hydroxyethoxyethylmethacrylat, Hydroxydiethoxyethylmethacrylat, Methoxyethylmethacrylat, Methoxyethoxyethylmethacrylat, Methoxydiethoxyethylmethacrylat, Poly(ethylenglykol)methacrylat, Methoxy-poly(ethylenglykol)methacrylat, Methacrylsäure, Natriummethacrylat, Glyzerinmethacrylat, Hydroxypropylmethacrylat, N-Vinylpyrrolidion und Hydroxybutylmethacrylat.

15. Matrixgesteuertes Arzneistoffdiffusionsabgabesystem gemäß Anspruch 1 oder 2 zur Verwendung bei einem Verfahren, umfassend:
Erzeugen eines Schnittes in einem Auge und
Implantieren des matrixgesteuerten Arzneistoffdiffusionsabgabesystems in diesem Auge durch den Schnitt.

## Revendications

1. Système de délivrance de médicament à diffusion régulée à matrice comprenant :
un copolymère de siloxane à chaîne latérale fluorée polymérisé avec une quantité thérapeutiquement efficace d'au moins un agent pharmaceutiquement actif et facultativement un ou plusieurs monomères.

2. Système de délivrance de médicament à diffusion régulée à matrice comprenant :
un copolymère de siloxane à chaîne latérale fluorée représenté par 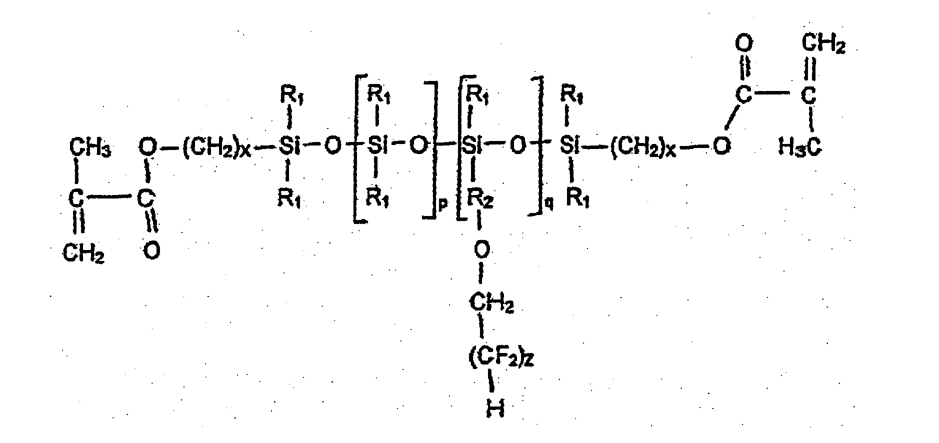
dans lequel les groupes R₁ peuvent être identiques ou différents et choisis dans le groupe constitué par un groupe alkyle en C_{1 à 7} et aryle en C_{6 à 10}; le groupe R₂ est un alkylène en C_{1 à 7} ; x est un nombre naturel inférieur à 26 ; p et q peuvent être des nombres naturels identiques ou différents inférieurs à 100 et z est un nombre naturel inférieur à 11, polymérisé avec une quantité thérapeutiquement efficace d'au moins un agent pharmaceutiquement actif et facultativement un ou plusieurs monomères.

3. Système de délivrance de médicament à diffusion régulée à matrice selon la revendication 1 ou 2, dans lequel ledit au moins un agent pharmaceutiquement actif est choisi dans le groupe constitué par les agents antiglaucomes, les agents anticataractes, les agents antirétinopathie diabétique, les agents thiols de réticulation, les agents anticancéreux, les modulateurs immunitaires, les agents anticoagulation, les agents anti-endommagement des tissus, les agents anti-inflammatoires, les agents antifibreux, les agents anti-inflammatoires non stéroïdiens, les antibiotiques, les agents antipathogènes, les dérivés de pipérazine, les agents cycloplégiques et les agents mydriatiques.

4. Système de délivrance de médicament à diffusion régulée à matrice selon la revendication 1 ou 2, dans lequel ledit au moins un agent pharmaceutiquement actif est choisi dans le groupe constitué par les anticholinergiques, les anticoagulants, les antifibrinolytiques, les antihistaminiques, les antipaludéens, les antitoxines, les agents chélatants, les hormones, les immunosuppresseurs, les thrombolytiques, les vitamines, les sels, les désensibilisateurs, les prostaglandines, les acides aminés, les métabolites et antiallergènes.

5. Système de délivrance de médicament à diffusion régulée à matrice selon la revendication 1 ou 2, dans lequel ledit au moins un agent pharmaceutiquement actif est choisi dans le groupe constitué par l'hydrocortisone, la gentamycine, le 5-fluorouracil, le sorbinil, l'interleukine 2, le phakan-a, la thioloa-thiopronine, le bendazac, l'acide acétylsalycilique, le fluocinolone acétonide, la trifluorothymidine, l'interféron, les modulateurs immunitaires et les facteurs de croissance.

6. Système de délivrance de médicament à diffusion régulée à matrice selon la revendication 1 ou 2, dans lequel lesdits un ou plusieurs monomères sont choisis dans le groupe constitué par le méthacrylate de méthyle, le N,N-diméthylacrylamide, l'acrylamide, le N-méthylacrylamide, le méthacrylate de 2-hydroxyéthyle, le méthacrylate d'hydroxyéthoxyéthyle, le méthacrylate d'hydroxydiéthoxyéthyle, le méthacrylate de méthoxyéthyle, le méthacrylate de méthoxyéthoxyéthyle, le méthacrylate de méthoxydiéthoxyéthyle, le méthacrylate de poly(éthylène glycol) le méthacrylate de méthoxy-poly(éthylène glycol), l'acide méthacrylique, le méthacrylate dé sodium, le méthacrylate de glycérol, le méthacrylate d'hydroxypropyle, la N-vinylpyrrolidione et le méthacrylate d'hydroxybutyle.

7. Copolymère de siloxane à chaîne latérale fluorée comprenant : 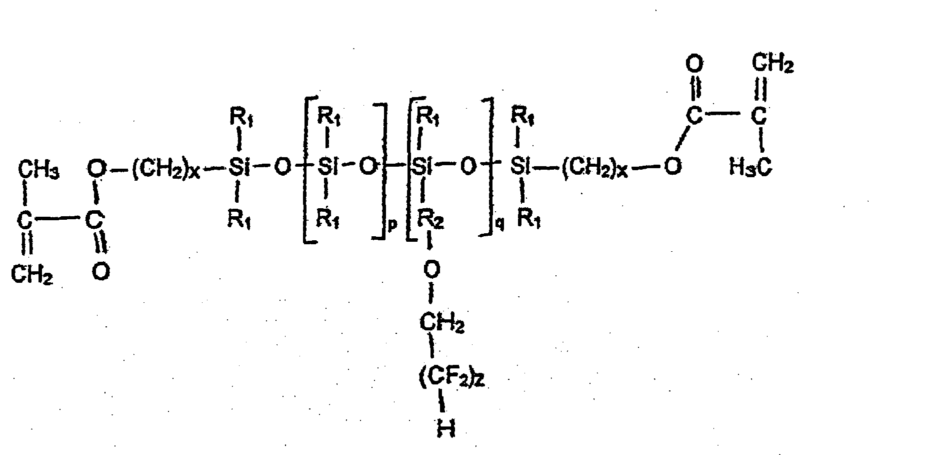 dans lequel les groupes R₁ peuvent être identiques ou différents et choisis dans le groupe constitué par un groupe alkyle en C_{1 à 7} et aryle en C_{6 à 10} ; le groupe R₂ est un alkylène en C_{1 à 7} ; x est un nombre naturel inférieur à 26 ; p et q peuvent être des nombres naturels identiques ou différents inférieurs à 100 et z est un nombre naturel inférieur à 11, à condition que les composés de formule suivante soit exclus : 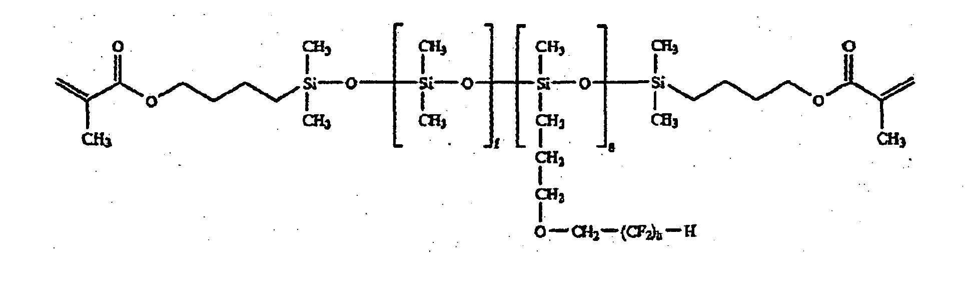 dans laquelle f et g sont des nombres naturels inférieurs à 100 ; et h est un nombre naturel inférieur à 11.

8. Procédé de fabrication d'un système de délivrance de médicament à diffusion régulée à matrice comprenant :
la polymérisation d'un copolymère de siloxane à chaîne latérale fluorée avec une quantité thérapeutiquement efficace d'au moins un agent pharmaceutiquement actif et facultativement un ou plusieurs monomères.

9. Procédé de fabrication d'un système de délivrance de médicament à diffusion régulée à matrice comprenant :
la polymérisation d'un copolymère de siloxane à chaîne latérale fluorée représenté par 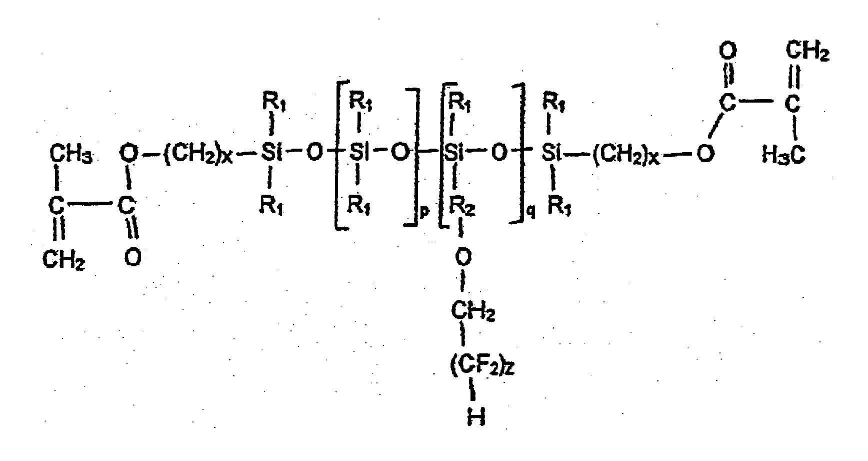
dans lequel les groupes R₁ peuvent être identiques ou différents et choisis dans le groupe constitué par un groupe alkyle en C_{1 à 7} et aryle en C_{6 à 10} ; le groupe R₂ est un alkylène en C_{1 à 7} ; x est un nombre naturel inférieur à 26 ; p et q peuvent être des nombres naturels identiques ou différents inférieurs à 100 et z est un nombre naturel inférieur à 11, avec une quantité thérapeutiquement efficace d'au moins un agent pharmaceutiquement actif et facultativement un ou plusieurs monomères.

10. Procédé de fabrication d'un système de délivrance de médicament à diffusion régulée à matrice comprenant les étapes consistant à :
préparer un siloxane coiffé de méthacrylate avec un copolymère à chaîne latérale perfluorée ;
copolymériser ledit siloxane coiffé de méthacrylate avec un copolymère à chaîne latérale perfluorée avec un ou plusieurs monomères et une quantité thérapeutiquement efficace d'au moins un agent pharmaceutiquement actif.

11. Procédé selon la revendication 8, 9 ou 10, dans lequel ledit au moins un agent pharmaceutiquement actif est choisi dans le groupe constitué par les agents antiglaucomes, les agents anticataracte, les agents antirétinopathie diabétique, les agents thiols de réticulation, les agents anticancéreux, les modulateurs immunitaires, les agents anticoagulation, les agents anti-endommagement des tissus, les agents anti-inflammatoires, les agents antifibreux, les agents anti-inflammatoires non stéroïdiens, les antibiotiques, les agents antipathogènes, les dérivés de pipérazine, les agents cycloplégiques et les agents mydriatiques.

12. Procédé selon la revendication 8, 9 ou 10, dans lequel ledit au moins un agent pharmaceutiquement actif est choisi dans le groupe constitué par les anticholinergiques, les anticoagulants, les antifibrinolytiques, les antihistaminiques, les antipaludéens, les antitoxines, les agents chélatants, les hormones, les immunosuppresseurs, les trombolytiques, les vitamines, les sels, les désensibilisateurs, les prostaglandines, les acides aminés, les métabolites et les antiallergènes.

13. Procédé selon la revendication 8, 9 ou 10, dans lequel ledit au moins un agent pharmaceutiquement actif est choisi dans le groupe constitué par l'hydrocortisone, la gentamycine, le 5-fluorouracil, le sorbinil, l'interleukine 2, le phakan-a, la thioloa-thiopronine, le bendazac, l'acide acétylsalicylique, le fluocinolone acétonide, la trifluorothymidine, l'interféron, les modulateurs immunitaires et les facteurs de croissance.

14. Procédé selon la revendication 8, 9 ou 10, dans lequel lesdits un ou plusieurs monomères sont choisis dans le groupe constitué par le méthacrylate de méthyle, le N,N-diméthylacrylamide, l'acrylamide, le N-méthylacrylamide, le méthacrylate de 2-hydroxyéthyle, le méthacrylate d'hydroxyéthoxyéthyle, le méthacrylate d'hydroxydiéthoxyéthyle, le méthacrylate de méthoxyéthyle, le méthacrylate de méthoxyéthoxyéthyle, le méthacrylate de méthoxydiéthoxyéthyle, le méthacrylate de poly(éthylène glycol), le méthacrylate de méthoxy-poly(éthylène glycol) l'acide méthacrylique, le méthacrylate de sodium, le méthacrylate de glycérol, le méthacrylate d'hydroxypropyle, la N-vinylpyrrolidione et le méthacrylate d'hydroxybutyle.

15. Système de délivrance de médicament à diffusion régulée à matrice selon la revendication 1 ou 2 pour une utilisation dans un procédé comprenant les étapes consistant à :
créer une incision au sein d'un oeil et
implanter ledit système de délivrance de médicament à diffusion régulée à matrice au sein dudit oeil à travers ladite incision.
